**Europäisches Patentamt**

**European Patent Office**

**Office européen des brevets**

(11) Publication number: **0 167 215**
**B1**

## EUROPEAN PATENT SPECIFICATION

(45) Date of publication of patent specification: **09.05.90**

(21) Application number: **85201085.9**

(22) Date of filing: **04.07.85**

(51) Int. Cl.⁵: **C 07 C 1/04, B 01 J 23/74,**
**B 01 J 23/86, C 10 G 47/14**

(54) Process for the preparation of hydrocarbons.

(30) Priority: **06.07.84 NL 8402149**

(43) Date of publication of application:
**08.01.86 Bulletin 86/02**

(45) Publication of the grant of the patent:
**09.05.90 Bulletin 90/19**

(84) Designated Contracting States:
**AT BE DE FR IT NL SE**

(56) References cited:
**EP-A-0 104 672**
**EP-A-0 109 702**
**GB-A-2 146 350**

**The file contains technical information**
**submitted after the application was filed and**
**not included in this specification**

(73) Proprietor: **SHELL INTERNATIONALE**
**RESEARCH MAATSCHAPPIJ B.V.**
**Carel van Bylandtlaan 30**
**NL-2596 HR Den Haag (NL)**

(72) Inventor: **Post, Martin Franciscus Maria**
**Badhuisweg 3**
**NL-1031 CM Amsterdam (NL)**
Inventor: **Sie, Swan Tiong**
**Badhuisweg 3**
**NL-1031 CM Amsterdam (NL)**

(74) Representative: **Aalbers, Onno et al**
**P.O. Box 302**
**NL-2501 CH The Hague (NL)**

Courier Press, Leamington Spa, England.

## Description

The invention relates to a process for the preparation of hydrocarbons by catalytic reaction of carbon monoxide with hydrogen.

The preparation of hydrocarbons from an $H_2/CO$ mixture by contacting this mixture at elevated temperature and pressure with a catalyst is known in the literature as the Fischer-Tropsch hydrocarbon synthesis process. Catalysts frequently used for this purpose contain one or more metals of the iron group together with one or more promoters and a carrier. For the preparation of these catalysts the known preparation techniques such as precipitation, impregnation, kneading and smelting are suitable. The products which can be prepared with these catalysts usually possess a very wide molecular weight distribution and in addition to branched and unbranched paraffins often contain considerable quantities of olefins and oxygen-containing organic compounds. As a rule only a minor portion of the resultant products consists of middle distillates. Besides the yield, the pour point of these middle distillates also leaves something to be desired. In view of this, the direct conversion of $H_2/CO$ mixtures by the Fischer-Tropsch process is not a particularly attractive route for the preparation of middle distillates on the technical scale.

In this patent application, the term "middle distillates" is understood as hydrocarbon mixtures having a boiling range principally corresponding to that of the kerosine and gas oil fractions which are obtained in the conventional atmospheric distillation of crude petroleum. The middle distillate range extends principally between approx. 150 and 360°C.

Recently a class of Fischer-Tropsch catalysts was discovered which possess the property of yielding a product containing only very few olefins and oxygen-containing organic compounds and consisting virtually completely of unbranched paraffins a substantial proportion of which have a boiling point above the middle distillate range. It has been found that the high-boiling portion of this product can be converted at high yield into middle distillates by means of hydrocracking. The feedstock chosen for the hydrocracking treatment is at least that portion of the product of which the initial boiling point is above the final boiling point of the heaviest middle distillate desired as end product. The hydrocracking treatment, which features a very low hydrogen consumption, yields middle distillates with a considerably better pour point than those obtained in the direct conversion of an $H_2/CO$ mixture by the Fischer-Tropsch process.

Fischer-Tropsch catalysts belonging to the abovementioned class contain silica as carrier and cobalt together with zirconium as catalytically active metals in such proportions that the catalysts contain 3—60 parts by weight of cobalt and 0.1—100 parts by weight of zirconium per 100 parts by weight of silica. The catalysts are prepared by applying the metals concerned to the silica by means of impregnation. For further information concerning the preparation of these catalysts by impregnation reference is made to European patent No. 127 220 in the name of the Applicant.

In European patent application 109 702 a process for the preparation of hydrocarbons has been described by catalytic reaction of hydrogen and carbon monoxide. A mixture of hydrogen and carbon monoxide is contacted with a catalyst present in a fixed bed, which catalyst comprises 5—40 pbw cobalt and 0.1—150 pbw of zirconium per 100 pbw of silica carrier, and which has been prepared by impregnating the carrier with compounds of cobalt and zirconium, followed by calcination and reduction of the obtained compositions. It has been described in this application that an improved $C_3{}^+$-selectivity is obtained when in the $H_2/CO$ feed a quantity of 10—40%v calculated on the overall mixture of steam is present.

A further investigation into the use of above-mentioned cobalt catalysts in the form of a fixed bed has now disclosed that their performance in terms of $C_5{}^+$ selectivity is in large measure dependent on the external and internal surface area of the catalyst bed.

It has been found that an optimum performance in terms of $C_5{}^+$ selectivity can be obtained if the catalyst has an external surface area ($S_e$) between 10 and 50 cm²/ml and an internal surface area ($S_i$) between 15 and 200 m²/ml such as to satisfy the relationship $10^6 > S_e{}^2 \times S_i > 2.5 \times 10^4$.

The present patent application therefore relates to a process for the preparation of hydrocarbons by catalytic reaction of carbon monoxide with hydrogen, in which a mixture of carbon monoxide and hydrogen is contacted at elevated temperature and pressure with a catalyst which contains 3—60 parts by weight of cobalt and 0.1—100 parts by weight of zirconium per 100 parts by weight of silica, and which catalyst is prepared by impregnation, the catalyst being present in the form of a fixed bed and the catalyst bed having an external surface area ($S_e$) between 10 and 50 cm²/ml and an internal surface area ($S_i$) between 15 and 200 m²/ml such as to satisfy the relationship $10^6 > S_e{}^2 \times S_i > 2.5 \times 10^4$.

The external surface area ($S_e$) of the catalyst bed can be found for a representative sample with a given volume expressed in ml by determining the external surface area expressed in cm² of each of the catalyst particles present therein, summing the external surface areas found and dividing the sum by the volume of the sample. The internal surface area $S_i$ of the catalyst bed is determined by means of nitrogen adsorption.

In the process according to the invention, use is preferably made of the cobalt catalysts which are the subject of European patent No. 127 220. These are catalysts which satisfy the relationship

$$3 > \frac{L}{S_i} > 0.3,$$

2

wherein

L = the total quantity of cobalt present on the catalyst, expressed in mg of Co/ml, and

$S_l$ = the internal surface area of the catalyst, expressed in m²/ml.

In the process according to the invention, preferential use is further made of cobalt catalysts prepared by any one of the two following procedures:

a) cobalt is first applied by impregnation in one or more steps and subsequently zirconium is likewise applied by impregnation in one or more steps, and

b) zirconium is first applied by impregnation in one or more steps and subsequently cobalt is likewise applied by impregnation in one or more steps.

In the process according to the invention, preferential use is further made of cobalt catalysts containing 15—50 parts by weight of cobalt per 100 parts by weight of silica. The quantity of zirconium preferentially present in the cobalt catalysts depends on the manner in which zirconium is applied. In the case of catalysts where first the cobalt and then zirconium is applied to the silica, preference is given to catalysts containing 0.1—5 parts by weight of zirconium per 100 parts by weight of silica. In the case of catalysts where first zirconium and then the cobalt is applied to the silica preference is given to catalysts containing from 5 to 40 parts by weight of zirconium per 100 parts by weight of silica.

According to the invention in order to achieve optimum performance in terms of $C_5^+$ selectivity, the $S_e$ and $S_l$ of the catalyst bed must satisfy the following requirements:

$$S_e = 10\text{—}50 \text{ cm}^2/\text{ml}$$

$$S_l = 15\text{—}200 \text{ m}^2/\text{ml, and}$$

$$10^6 > S_e^2 \times S_l > 2.5 \times 10^4.$$

It is preferred to use a catalyst bed which satisfies:

$$2.5 \times 10^5 > S_e^2 \times S_l > 3 \times 10^4.$$

Before becoming suitable for utilization in the preparation of hydrocarbons from an $H_2$/CO mixture, the cobalt catalysts have to be activated. This activation can suitably be carried out by contacting the catalysts at a temperature between 200 and 350°C with hydrogen or a hydrogen-containing gas.

The conversion of the $H_2$/CO mixture into hydrocarbons according to the invention is preferentially carried out at a temperature of 125 to 350°C, and in particular of 175 to 275°C, and a pressure of 5 to 500 bar, in particular of 10 to 75 bar.

$H_2$/CO mixtures which are suitable for conversion according to the invention into hydrocarbons can be very suitably obtained starting from light hydrocarbons such as methane by means of steam reforming or partial oxidation. Special preference is given to the use of natural gas as feedstock for the preparation of the $H_2$/CO mixture.

. The $H_2$/CO mixture converted according to the invention into hydrocarbons preferentially possesses an $H_2$/CO molar ratio of more than 1.5. If the feedstock possesses an $H_2$/CO molar ratio of less than 1.5, it is preferentially raised to a value between 1.5 and 2.5 and in particular to a value between 1.75 and 2.25 before being contacted with the cobalt catalyst. Raising of the $H_2$/CO molar ratio of hydrogen-lean $H_2$/CO mixtures can be performed, inter alia, by the addition of hydrogen, removal of carbon monoxide, admixture of a hydrogen-rich $H_2$/CO mixture or by applying the CO shift reaction to the hydrogen-lean $H_2$/CO mixture.

As has already been mentioned above, when used for the conversion of an $H_2$/CO mixture the present cobalt catalysts yield a substantially paraffinic product of which the high-boiling portion can be converted at high yield into middle distillates by means of a hydrocracking treatment. The feedstock chosen for the hydrocracking treatment is at least that portion of the product of which the initial boiling point is above the final boiling point of the heaviest middle distillate desired as end product.

Although in the preparation of middle distillates the product obtained over the cobalt catalyst it is sufficient to use as feedstock for the hydrocracking treatment that portion of the product of which the initial boiling point is above the final boiling point of the heaviest middle distillate desired as end product, for this purpose it is preferred to use the total $C_5^+$ fraction of the product prepared over the cobalt catalyst because it has been found that under the influence of the catalytic hydrogen treatment a quality improvement takes place in the gasoline, kerosine and gas oil fractions present therein.

The hydrocracking treatment is carried out by contacting the fraction to be treated, at elevated temperature and pressure and in the presence of hydrogen, with a catalyst containing one or more noble metals of Group VIII on a carrier. The hydrocracking catalyst used is preferentially a catalyst containing 0.1—2% by weight and in particular 0.2—1% by weight of one or more noble metals of Group VIII on a carrier. Preference is given to catalysts containing as Group VIII noble metal platinum or palladium, and silica-alumina as carrier. The hydrocracking treatment is preferentially carried out at a temperature of 200—400°C and in particular of 250—350°C and a pressure of 5—200 bar and in particular of 10—75 bar.

The invention will now be elucidated with reference to the following Example.

Example

Nine $Co/Zr/SiO_2$ catalysts (catalysts 1—9) were prepared by impregnation of six spherical silica carriers (silicas A—F) with solutions of cobalt and zirconium compounds. At each impregnation step a quantity of solution was used of which the volume substantially corresponded to the pore volume of the carrier concerned. After each impregnation step the solvent was removed by heating and the material was calcined at 500°C. After the final calcination the compositions were activated in hydrogen as follows: catalysts 1—3, 6, 8 and 9 at 260°C and catalysts 4, 5 and 7 at 250°C. Catalysts 1—9 were prepared as follows.

Catalysts 1, 2, 3, 6, 8 and 9

One-step or multi-step impregnation of a silica carrier with a solution of zirconium tetra-n-propoxide in a mixture of n-propanol, toluene and acetyl acetone, followed by one-step impregnation of the zirconium-loaded carrier with an aqueous solution of cobalt nitrate. In the preparation of catalysts 1 and 3, the zirconium impregnation was carried out in three steps; catalysts 6, 8 and 9 were prepared via two-step zirconium impregnation; in the preparation of catalyst 2, zirconium impregnation took place in one step.

Catalysts 4, 5 and 7

One-step impregnation of a silica carrier with an aqueous solution of cobalt nitrate, followed by one-step impregnation of the cobalt-loaded carrier with an aqueous solutin of zirconium nitrate.

Further particulars of catalysts 1—9 are shown in Table I.

Catalysts 1—9 were used in nine experiments (experiments 1—9) in the preparation of hydrocarbons from a mixture of carbon monoxide and hydrogen having an $H_2/CO$ molar ratio of 2. The experiments were carried out at a pressure of 20 bar in a reactor in which there was a fixed catalyst bed. The other conditions under which these experiments were carried out, together with the results of the experiments, are shown in Table II.

Of the experiments shown in Table II, only experiments 7—9 are experiments according to the invention. In these experiments the catalyst bed satisfied the relationship $10^6 > S_e^2 \times S_l > 2.5 \times 10^4$ and a high $C_5^+$ selectivity was achieved. Experiments 1—6 come outside the scope of the invention. They are included in the patent application for purposes of comparison. In these experiments using a catalyst bed satisfying $S_e^2 \times S_l < 2.5 \times 10^4$, a relatively low $C_5^+$ selectivity was observed.

TABLE I

| Catalyst No. | 1 | 2 | 3 | 4 | 5 | 6 | 7 | 8 | 9 |
|---|---|---|---|---|---|---|---|---|---|
| Silica carrier | A | B | B | A | C | C | D | E | F |
| Zr load, ppw Zr/100 ppw $SiO_2$ | 18 | 6 | 18 | 0.9 | 0.9 | 12 | 0.9 | 12 | 12 |
| Co load, mg Co/ml catalyst | 41 | 33 | 101 | 36 | 93 | 102 | 97 | 94 | 40 |
| $S_i$, $m^2$/ml | 28 | 32 | 30 | 29 | 107 | 104 | 100 | 97 | 31 |
| $S_e$, $cm^2$/ml | 15 | 26 | 26 | 15 | 14 | 14 | 26 | 24 | 35 |
| $S_e^2 \times S_i$ | 6300 | 21632 | 20280 | 6525 | 20972 | 20384 | 67600 | 55872 | 40176 |

EP 0 167 215 B1

TABLE II

| Experiment No. | 1 | 2 | 3 | 4 | 5 | 6 | 7 | 8 | 9 |
|---|---|---|---|---|---|---|---|---|---|
| Catalyst No. | 1 | 2 | 3 | 4 | 5 | 6 | 7 | 8 | 9 |
| Temperature, °C | 240 | 227 | 220 | 240 | 225 | 215 | 215 | 210 | 220 |
| Space velocity, $Nl.l^{-1}.hour^{-1}$ | 1000 | 900 | 1000 | 1000 | 900 | 900 | 900 | 900 | 900 |
| Conversion of the $H_2/CO$ mixture, % by volume | 57 | 60 | 53 | 52 | 64 | 65 | 61 | 58 | 63 |
| $C_1^+$ production, $g.l^{-1}.hour^{-1}$ | 108 | 101 | 103 | 100 | 109 | 110 | 103 | 101 | 110 |
| $C_5^+$ selectivity, % by weight | 60 | 72 | 64 | 52 | 64 | 70 | 76 | 79 | 78 |

EP 0 167 215 B1

## Claims

1. Process for the preparation of hydrocarbons by catalytic reaction of carbon monoxide with hydrogen, characterized in that a mixture of carbon monoxide and hydrogen is contacted at elevated temperature and pressure with a catalyst which contains 3—60 parts by weight of cobalt and 0.1—100 parts by weight of zirconium per 100 parts by weight of silica, the catalyst being prepared by impregnation, in that the catalyst is present in the form of a fixed bed and in that the catalyst bed has an external surface area ($S_e$) between 10 and 50 $cm^2/ml$ and an internal surface ($S_i$) between 15 and 200 $m^2/ml$ such as to satisfy the relationship $10^6 > S_e^2 \times S_i > 2.5 \times 10^4$.

2. Process as claimed in claim 1, characterized in that the catalyst bed has an $S_e$ and an $S_i$ such as to satisfy the relationship $2.5 \times 10^5 > S_e^2 \times S_i > 3 \times 10^4$.

3. Process as claimed in claim 1 or 2, characterized in that the catalyst satisfies the relationship

$$3 > \frac{L}{S_i} > 0.3,$$

wherein

L = the total quantity of cobalt present on the catalyst, expressed in mg of Co/ml, and
$S_i$ = the internal surface area of the catalyst, expressed in $m^2/ml$.

4. Process as claimed in any one of claims 1—3, characterized in that the catalyst contains 15—50 parts by weight of cobalt per 100 parts by weight of silica and either 0.1—5 parts by weight of zirconium if in the preparation first cobalt and then zirconium is applied or 5—40 parts by weight of zirconium if in the preparation first zirconium and then cobalt is applied.

5. Process as claimed in any one of claims 1—4, characterized in that it is carried out at a temperature of 125—350°C and a pressure of 5—100 bar.

6. Process as claimed in any one of claims 1—5, characterized in that the $H_2/CO$ mixture has an $H_2/CO$ molar ratio between 1.75 and 2.25.

7. Process as claimed in any one of claims 1—6, characterized in that in order to prepare middle distillates from the product prepared over the cobalt catalyst, at least that portion of which the initial boiling point is above the final boiling point of the heaviest middle distillate desired as end product is subjected to a hydrocracking treatment by contacting it at elevated temperature and pressure with a catalyst containing one or more noble metals of Group VIII on a carrier.

8. Process as claimed in claim 7, characterized in that the hydrocracking treatment a catalyst is used containing 0.1—2% by weight of one or more noble metals of Group VIII.

9. Process as claimed in any one of claims 7—8, characterized in that in the hydrocracking treatment a catalyst is used containing platinum or palladium as noble metal of Group VIII and silica-alumina as carrier.

## Patentansprüche

1. Verfahren zur Herstellung von Kohlenwasserstoffen durch katalytische Umsetzung von Kohlenmonoid mit Wasserstoff, dadurch gekennzeichnet, daß ein Gemisch von Kohlenmonoxid mit Wasserstoff bei erhöhter Temperatur und bei erhöhtem Druck mit einem Katalysator in Berührung gebracht wird, der 3 bis 60 Gewichtsteile Kobalt und 0,1 bis 100 Gewichtsteile Zirkon je 100 Gewichtsteile Siliciumdioxid enthält, wobei der Katalysator durch Imprägnieren hergestellt worden ist, daß der Katalysator in der Form eines Festbettes vorliegt und daß das Katalysatorbett eine solche äußere Oberfläche ($S_e$) von 10 bis 50 $cm^2/ml$ und eine solche inner Oberfläche ($S_i$) von 15 bis 200 $m^2/ml$ aufweist, daß die Beziehung $10^6 > S_e^2 \times S_i > 2,5 \times 10^4$ erfüllt wird.

2. Verfahren nach Anspruch 1, dadurch gekennzeichnet, daß das Katalysatorbett eine solchen $S_e$-Wert und einen solchen $S_i$-Wert aufweist, daß die Beziehung $2,5 \times 10^5 > S_e^2 \times S_i > 3 \times 10^4$ erfüllt wird.

3. Verfahren nach Anspruch 1 oder 2, dadurch gekennzeichnet, daß der Katalysator der Beziehung

$$3 > \frac{L}{S_i} > 0,3$$

entspricht, worin

L = Gesamtmenge an Kobalt, die auf dem Katalysator vorliegt, ausgedrückt in mg CO/ml, und
$S_i$ = innere Oberfläche des Katalysators, ausgedrückt in $m^2/ml$.

4. Verfahren nach einem der Ansprüche 1 bis 3, dadurch gekennzeichnet, daß der Katalysator 15 bis 50 Gewichsteile Kobalt je 100 Gewichtsteile Siliciumdioxid und entweder 0,1 bis 5 Gewichtsteile Zirkon, wenn in der Herstellung des Katalysators zunächst Kobalt und dann Zirkon aufgebracht wird, oder 5 bis 40 Gewichtsteile Zirkon, wenn in der Herstellung zunächst Zirkon und dann Kobalt aufgebracht wird, enthält.

5. Verfahren nach einem der Ansprüche 1 bis 4, dadurch gekennzeichnet, daß es bei einer Temperatur von 125—350°C und bei einem Druck von 5—100 bar ausgeführt wird.

6. Verfahren nach einem der Ansprüche 1 bis 5, dadurch gekennzeichnet, daß das $H_2$/CO-Gemisch ein $H_2$/CO-Molverhältnis zwischen 1,75 und 2,25 aufweist.

7. Verfahren nach einem der Ansprüche 1 bis 6, dadurch gekennzeichnet, daß zur Herstellung von Mitteldestillaten aus dem über dem Kobaltkatalysator hergestellten Produkt zumindest jener Teil des Produktes, dessen Anfangssiedepunkt oberhalb des Endsiedepunktes des als Endprodukt gewünschten schwersten Mitteldestillates liegt, einem Hydrocracken unterzogen wird, indem er bei erhöhter Temperatur und bei erhöhtem Druck mit einem Katalysator in Berührung gebracht wird, der ein oder mehrere Edelmetalle de Gruppe VIII, aufgebracht auf einem Träger, umfaßt.

8. Verfahren nach Anspruch 7, dadurch gekennzeichnet, daß im Hydrocracken ein Katalysator eingesetzt wird, der 0,1—2 Gew.-% eines oder mehrerer Edelmetalle aus der Gruppe VIII enthält.

9. Verfahren nach einem der Ansprüche 7 und 8, dadurch gekennzeichnet, daß in der Hydrocrackbehandlung ein Katalysator verwendet wird, der Platin oder Palladium als Edelmetall aus der Gruppe VIII und Siliciumdioxid-Aluminiumoxid als Trägermaterial enthält.

**Revendications**

1. Procédé pour la préparation catalytique d'hydrocarbures par réaction catalytique d'oxyde de carbone avec l'hydrogène, caractérisé en ce qu'un mélange d'oxyde de carbone et d'hydrogène est mis en contact à température et pression élevées avec un catalyseur qui contient 3—60 parties en poids de cobalt et 0,1—100 parties en poids de zirconium pour 100 parties en poids de silice, le catalyseur étant préparé par imprégnation, en ce que le catalyseur est présent sous la forme d'un lit fixe et en ce que le catalyseur a une aire superficielle extérieure ($S_e$) comprise entre 10 et 50 cm²/ml et une aire superficielle intérieure ($S_i$) comprise entre 15 et 200 m²/ml de manière à satisfaire à la relation $10^6 > S_e^2 \times S_i > 2,5 \times 10^4$.

2. Procédé selon la revendication 1, caractérisé en ce que le lit de catalyseur a des valeurs de $S_e$ et $S_i$ telles que la relation suivante soit satisfaite: $2,5 \times 10^5 > S_e^2 \times S_i > 3 \times 10^4$.

3. Procédé selon la revendication 1 où 2, caractérisé en ce que le catalyseur satisfait à la relation

$$3 > \frac{L}{S_i} > 0,3,$$

où

L = la quantité totale de cobalt présente sur le catalyseur, exprimée en mg de Co par ml et
$S_i$ = l'aire superficielle intérieure du catalyseur, exprimée en m²/ml.

4. Procédé selon l'une quelconque des revendications 1—3, caractérisé en ce que le catalyseur contient 15—50 parties en poids de cobalt pour 100 parties en poids de silice et soit 0,1—5 parties en poids de zirconium si dans la préparation le cobalt est appliqué d'abord et ensuite le zirconium, soit 5—40 parties en poids de zirconium si dans la préparation le zirconium est appliqué d'abord et ensuite le cobalt.

5. Procédé selon l'une quelconque des revendications 1—4, caractérisé en ce qu'il est mis en oeuvre à une température de 125—350°C et une pression de 5—100 bars.

6. Procédé selon l'une quelconque des revendications 1—5, caractérisé en ce que le mélange $H_2$/CO a un rapport molaire $H_2$/CO compris entre 1,75 et 2,25.

7. Procédé selon l'une quelconque des revendications 1—6, caractérisé en ce que pour préparer des distillats moyens à partir du produit préparé sur le catalyseur au cobalt, au moins la portion dont le point initial d'ébullition se trouve au-dessus du point final d'ébullition du distillat moyen le plus lourd désiré comme produit final est soumise à un traitement d'hydrocraquage par mise en contact à température et pression élevées avec un catalyseur contenant un ou plusieurs métaux nobles du groupe VIII sur un support.

8. Procédé selon la revendication 7, caractérisé en ce que dans le traitement d'hydrocraquage on utilise un catalyseur contenant 0,1—2% en poids d'un ou plusieurs métaux nobles du groupe VIII.

9. Procédé selon l'une quelconque des revendications 7—8, caractérisé en ce que dans le traitement d'hydrocraquage on utilise un catalyseur contenant du platine ou du palladium comme métal noble du groupe VIII et de la silice-alumine comme support.